(19) Europäisches Patentamt
European Patent Office
Office européen des brevets

(11) Publication number: **0 239 063 A2**

(12) **EUROPEAN PATENT APPLICATION**

(21) Application number: 87104292.5

(22) Date of filing: 24.03.87

(51) Int. Cl.⁴: C 07 C 102/06, C 07 C 103/46

(30) Priority: 27.03.86 JP 68969/86

(43) Date of publication of application: 30.09.87 Bulletin 87/40

(84) Designated Contracting States: BE CH DE ES FR GB IT LI NL SE

(71) Applicant: KANEGAFUCHI KAGAKU KOGYO KABUSHIKI KAISHA, 2-4 Nakanoshima 3-chome, Kita-ku Osaka-shi Osaka-fu (JP)

(72) Inventor: Takahashi, Satomi, 1-13-13, Shinwadai, Tarumi-ku, Kobe-shi, Hyogo-ken (JP)
Inventor: Ohashi, Takehisa, 9-14, Shinoharaobanoyama-cho 3-chome, Nada-ku, Kobe-shi, Hyogo-ken (JP)
Inventor: Watanabe, Kiyoshi, 15-41, Matsugaoka 5-chome,, Akashi-shi, Hyogo-ken (JP)

(74) Representative: Türk, Gille, Hrabal, Bruckner Strasse 20, D-4000 Düsseldorf 13 (DE)

(54) **Process for preparing N-omega-trifluoroacetyl amino acid.**

(57) A process for preparing an $N^{\omega}$-trifluoroacetyl amino acid which comprises reacting a trifluoroacetic acid ester with a basic amino acid in an aqueous medium under an alkali condition. According to the process, $N^{\omega}$-trifluoroacetyl amino acid, particularly $N^{\omega}$-trifluoroacetyl derivatives of lysine and ornithine, can be economically, easily and efficiently prepared in industrial scale.

EP 0 239 063 A2

# PROCESS FOR PREPARING $N^{\omega}$-TRIFLUOROACETYL AMINO ACID

The present invention relates to a process for preparing an $N^{\omega}$-trifluoroacetyl amino acid which comprises selectively introducing trifluoroacetyl group as a protective group into only amino group at $\omega$-position of a basic amino acid (hereinafter reffered to as "$N^{\omega}$"), and more particularly to a process for advantageously preparing $N^{\omega}$-trifluoroacetyl derivatives of lysine and ornithine useful as starting materials for physiolocically active peptide in industrial scale.

As a method for introducing trifluoroacetyl group into an $\alpha$-amino acid, there have been known various method such as a method using trifluoroacetyl acid anhydride [Chemische Berichte (Chem. Ber.), 89, 647 (1956)], a method using S-ethyl trifluorothioacetate in an aqueous medium [Journal of the American Chemical Society (J. Am. Chem. Soc.), 77, 2779 (1955)], a method using phenyl trifluoroacetate in phenol [Chem. Ber., 92, 2095 (1959)], a method using an sym-trichloro trifluoro acetate in dimethylsulfoxide (DMSO) [The Journal of Organic Chemistry (J. Org. Chem), 35, 4275 (1970)], and a method using ethyl trifluoroacetate in dried methanol [J. Org. Chem., 44, 2805 (1979)], but as a method for preparing $N^{\omega}$-trifluoroacetyl amino acid which comprises selectively introducing trifluoroacetyl group into amino group at $\omega$-position of basic amino acid ($N^{\omega}$), there is only known a method using S-ethyl trifluorothioacetate as shown by the following reaction formula.

$$CF_3COSC_2H_5 + NH_2\text{-}\underset{}{CH}(\text{-}(CH_2)_n\text{-}NH_2)\text{-}COOH \longrightarrow$$

$$NH_2\text{-}CH((CH_2)_n\text{-}NHCOCF_3)\text{-}COOH \quad + \quad C_2H_5\text{-}SH$$

wherein n is 3 or 4. Accordingly, when the $N^{\omega}$-trifluoroacetyl derivatives of lysine or ornithine are prepared, only the method using S-ethyl trifluoroacetate have substantially carried out.

The method for preparing $N^{\omega}$-trifluoroacetyl amino acid, which uses S-ethyl trifluorothioacetate, has an advantage that only amino group at $\omega$-position of basic amino acid can be selectively and easily trifluoroacetylated without converting amino group at $\alpha$-position of the basic amino acid (hereinafter referred to as "$N^{\alpha}$-"). However, in the synthesis of S-ethyl trifluorothioacetate which is a starting material of the above reaction, there are problems that a large amount of trifluoroacetic acid anhydride which is relatively expensive is required, as shown by the following reaction formula:

$$(CF_3CO)_2O + C_2H_5\text{-}SH \longrightarrow$$

$$CF_3COSC_2H_5 + CF_3COOH$$

and that trifluoroacetic acid produced as a by-product cannot be effectively used. Further, during the synthesis of the starting material and trifluoroacetylation of amino group at $\omega$-position of basic amino acid, a troublesome operation and a specific step are required for inhibiting powerful stink due to ethyl mercaptan. As aforementioned, when $N^{\omega}$-trifluoroacetyl amino acid is industrially prepared, there are various defects in the operational and economic aspects.

In peptide synthesis, when benzyloxycarboxyl group or benzyl group, which is widely used as a typical protective group of the peptide, is eliminated, catalytic hydrogenation with paladium/carbon catalyst is conducted as a usual manner. However, in the synthesis of $N^{\omega}$-trifluoroacetyl amino acid, the paladium catalyst is easily inactivated by a very small amount of sulfur compound and the reaction is easy to fail. Accordingly,

the use of mercaptans in the preparation of protected amino acid is not preferable since there is the possibility that the mercaptans are incorporated into the resultant product as a disulfide and the like. From the above-mentioned viewpoint, the use of S-ethyl fluorothioacetate itself has defects in the purification of the reaction product, and the like.

Also, there has been reported the method in which trifluoroacetate is used in the organic solvent to prepare trifluoroacetyl derivatives of neutral or acidic amino acids, as mentioned above. However, when the trifluoroacetyl derivatives of basic amino acids are prepared under the conditions described in the above method, it is hardly observed that the amino group at $\omega$-position of the basic amino acid is selectively reacted with trifluoroacetyl group, and accordingly, most of the obtained product is $N^{\alpha}$,$N^{\omega}$-ditrifluoroacetyl derivative (the derivative being trifluoroacetylated at $\alpha$-position and $\omega$-position of the amino acid) and the rest is a mixture of $N^{\alpha}$-trifluoroacetyl derivative and $N^{\omega}$-trifluoroacetyl derivative in a very small amount. Therefore, it is difficult to practically produce $N^{\omega}$-trifluoroacetyl amino acid according to the method using trifluoroacetate in the organic solvent.

An object of the present invention is to provide a process for industrially preparing $N^{\omega}$-trifluoroacetyl amino acid which is economic, easy and effecient.

Another object of the present invention is to provide a process for industrially advantageously preparing $N^{\omega}$-trifluoroacetyl derivative of lysine and ornithine useful as staring materials for physiologically active peptide.

The above and other objects of the present invention will become apparent from the description hereinafter.

It has been now found that $N^{\omega}$-trifluoroacetyl amino acid in which trifluoroacetyl group is attached to only amino group at $\omega$-position of a basic amino acid can be extremely easily obtained by reacting a trifluoroacetic acid ester which can be easily prepared from inexpensive trifluoroacetic acid and an alcohol with a basic amino acid in an aqueous medium under alkali condition.

According to the present invention, there is provided a process for preparing $N^{\omega}$-trifluoroacetyl amino acid which comprises reacting a trifluoroacetic acid ester with a basic amino acid in an aqueous medium under alkali conditin.

The reaction formula of the present invention is as follows:

$$CF_3COOR + \overset{\displaystyle (CH_2)_n-NH_2}{NH_3^+-CH-COO^-} \longrightarrow$$

$$\overset{\displaystyle (CH_2)_n-NHCOCF_3}{NH_3^+-CH-COO^-} \quad + \quad R-OH$$

wherin R is an alkyl group, aralkyl group or aryl group and n is an integer of 1 to 6. That is, according to the present invention, trifluoroacetyl group is selectively introduced into the amino group at $\omega$-position of the basic amino acid by transtrifluoroacetylation of the trifluoroacetic acid ester to prepare $N^{\omega}$-trifluoroacetyl amino acid.

In the present invention, trifluoroacetic acid ester is reacted with the basic amino acid while adjusting the pH of the reaction system in the aqueous medium to a pH range that protonation is selectively conducted at the only amino group at $\alpha$-position of the basic amino acid and the amino group at $\omega$-position of the basic amino acid can be maintained in undissociated state, that is, from pH 8 to pH 11, thus resulting in

advantageous synthesis of Nω-trifluoroacetyl amino acid by utilizing the selective transtrifluoroacetylation at amino group at ω-position of the basic amino acid.

The trifluoroacetic acid ester used as a starting material can be easily prepared by heating trifluoroacetic acid and alcohols with refluxing to conduct esterification. Usually, the reaction may be carried out in the absence of a catalyst and it is preferable to use excess alcohols. Various alcohols can be used in the present invention. Examples of the alcohols are, for instance, an alkyl alcohol such as methanol, ethanol, n-propanol, iso-propanol, n-buthanol, iso-buthanol or sec-buthanol; an aralkyl alcohol such as benzyl alcohol or phenethyl alcohol; an aryl alcohol such as phenol or p-nitro phenol; and the like. Among them, it is preferable that the alkyl alcohols having 2 to 4 carbon aboms are used from the point of operation.

As the reaction solvent, it is essential to use the aqueous medium since in an organic solvent, it is actually difficult to ionize the functional group of basic amino acid selectively. It is effective to use water or a mixture of water and an alcohol.

The reaction temperature varies depending on kinds of trifluoroacetic acid ester used and cannot be specified generally. In case of using an alkyl ester of trifluoro acetic acid, the reaction can be usually carried out at a temperature of $0^{\circ}C$ to room temperature and it is not required to elevate the temperature. When the reaction is carried out at excessively high temperature, alkali hydrolysis of trifluoroacetic acid ester which is a side reaction occurs and the desired product is obtained in a low yield.

It is important that the reaction is carried out under alkali condition and it is preferable that the reaction is carried out within the range of pH 8 to pH 11 from the point of selective trifluoroacetylation of amino group at ω-position of the basic amino acid. An inorganic base such as a hydroxide of alkali metal or a

carbonate of alkali metal can be effectively used for controlling the pH of the reaction system.

In the present invention, trifluoroacetic acid ester is added to the basic amino acid in aqueous medium with stirring, and in the above reaction, it is possible to use the esterification reaction mixture of the trifluoroacetic acid and the alcohol without purifying or isolating. With proceeding of the reaction, $N^{\omega}$-trifluoro acetyl amino acid is deposited as a white precipitate. Accordingly, the precipitate is only filtered off from the reaction mixture and washed with water to give the desired product. The obtained crude product is satisfactorily high in purity, but, if necessary, the purified product can be easily obtained in a usual purifying operation such as recrystallization from water or a mixture of water and an alcohol.

The present invention is more specifically • described and explained by means of the following Examples and Comparative Examples in which all percentages and parts are by weight unless otherwise noted. It is to be understood that the present invention is not limited to the Examples, and various changes and modifications may be made in the invention without departing from the spirit and scope thereof.

Example 1

There was added 200 mℓ of 1N aqueous solution of sodium hydroxide to 36.5 g (0.20 mol) of L-lysine hydrochloride and the lysine was dissolved in the aqeuous solution with stirring (pH 10.5). The obtained aqueous solution of the lysine was cooled with ice to a temperature of 5°C, to which 42.6 g (0.30 mol) of ethyl trifluoroacetate was added at 5°C, and the mixture was vigorously stirred. After 3 to 4 minutes from the addition of ethyl trifluoroacetate, a white precipitate began to deposit and the reaction was continued for 3 hours by allowing it to stand. After adjusting the pH of the reaction mixture to pH 5.8, the white precipitate was

filtered off from the reaction mixture and washed with cool water and then ethanol, and was dried under reduced pressure to give 28.3 g of $N^{\varepsilon}$-trifluoroacetyl-L-lysine (a yield of $N^{\varepsilon}$-trifluoroacetyl-L-lysine to L-lisine hydrochloride: 58.5 %). The filtrate and the washing water were concentrated and 9.1 g of $N^{\varepsilon}$-trifluoroacetyl-L-lysine was obtained in the same manner as mentioned above.

The obtained $N^{\varepsilon}$-trifluoroacetyl-L-lysine was dissolved in 300 mℓ of hot water and a small amount of ethanol was added to the aqueous solution, which was allowed to stand overnight in a refrigerator to deposit white flaky crystals. The deposited crystals were filtered off and dried to give 26.5 g of $N^{\varepsilon}$-trifluoroacetyl-L-lysine in a pure state.

The obtained product had following properties.

$[\alpha]_D^{25}$= +18.6 (c=1, 1N-HCℓ)
m.p.: from 260° to 262°C (decomposition)

Example 2

To a solution (pH 10.5) prepared by adding 50 mℓ of 1N aqueous solution of sodium hydroxide to 8.43 g (50 mmol) of L-ornithine hydrochloride and dissolving the ornithine in the aqueous solution was added 10.65 g (75 mmol) of ethyl trifluoroacetate at a temperature of 5°C while cooling the aqueous solution of ornithine with ice, and the mixture was vigorously stirred for 2 hours. During the reaction, pH was maintained from 10 to 8 by adding 1N-NaOH. The deposited white precipitate was filtered off from the reaction mixture, washed with ethanol and dried under reduced pressure to give 5.90 g of $N^{\delta}$-trifluoroacetyl-Lornithine (a yield of $N^{\delta}$-trifluoroacetyl-L-ornithine to L-ornithine hydrochloride: 46.1 %). There was obtained 4.95 g of $N^{\delta}$-trifluoroacetyl L-ornithine in a pure state by recrystallizing from 50 mℓ of water.

The obtained product had following properties.

$[\alpha]_D^{25}$= +16.4 (c=1, 1N-HCℓ)
m.p.: from 247° to 249°C (decomposition)

Example 3

After refluxing 4.56 g (40 mmol) of trifluoroacetic acid and 4.8 g (80 mmol) of isopropyl alcohol in an oil bath having a temperature of 75°C for 2 hours with heating, the reaction mixture was cooled. A separating funnel was charged with the obtained reaction mixture, to which 30 mℓ of water was added and was allowed to stand. The under layer was taken out from the funnel to give isopropyl trifluoroacetate.

To a solution (pH 10.5) prepared by adding 20 mℓ of 1N aqueous solution of sodium hydroxide to 3.65 g (20 mmol) of L-lysine hydrochloride and stirring the mixture to dissolve was added 3.90 g (25 mmol) of the obtained isopropyl trifluoroacetate. The reaction was conducted at a room temperature for 4 hours by vigorously stirring. The produced white precipitate was filtered off from the reaction mixture, washed with ethanol and dried under reduced pressure to give 1.32 g of $N^{\varepsilon}$-trifluoroacetyl-L-lysine.

The obtained product had the same property as one in Example 1.

Example 4

The procedure of Example 3 was repeated except that n-butyl alcohol was used instead of isopropyl alcohol to give n-butyl trifluoroacetate. There was obtained 1.93 g of $N^{\varepsilon}$-trifluoroacetyl-L-lysine in the same manner as in Example 3 except that 4.75 g (25 mmol) of n-butyl trifluoroacetate was used instead of isopropyl trifluoroacetate.

The obtained product had the same property as one in Example 1.

Comparative Examples 1 and 2

There was suspended 1.82 g (10 mmol) of L-lysine

hyrochloride (L-Lys·HCℓ) in 5.0 mℓ of dried methanol which was previously dried with Molecular Sieves, to which 1.40 mℓ (10 mmol) (Comparative Example 1) or 2.80 mℓ (20 mmol) (Comparative Example 2) of triethylamine (TEA) was added and the mixture was stirred for 1 hour. To the reaction mixture was added 1.50 mℓ (12.5 mmol) of ethyl trifluoroacetate and the reaction was conducted at room temperature for 30 hours by vigorously stirring.

A part of the reaction mixture was sampled and was determined by using a high performance liquid chromatography (HPLC). Conditions for determining are as follows:

Column: Finepak SIL $C_{18-5}$ (made by Japan Spectroscopic Co., Ltd.) 4.6 mm ID x 250 mm
Mobile phase: 60 mM buffer solution of phosphoric acid (pH 2.5)/acetonitrile = 85/15(V/V)
Flow rate: 1.0 mℓ/minute
Detection: 210 nm

The results are shown in Table 1.

In addition to the ingredients used in the Examples, other ingredients can be used in the Examples as set forth in the specification to obtain substantially the same results.

Table 1

| Com.Ex. No. | L-Lys·HCℓ/TEA | Amount of $N^{\alpha}$-trifluoroacetyl-L-lysine (yield) | Amount of $N^{\varepsilon}$-trifluoroacetyl-L-lysine (yield) | Amount of $N^{\alpha},N^{\varepsilon}$-di-trifluoroacetyl-L-lysine (yield) |
|---|---|---|---|---|
| Com. Ex. 1 | 1/1 | 16 mg (0.7 %) | 45 mg (1.9 %) | 1.05 g (27 %) |
| Com. Ex. 2 | 1/2 | 74 mg (3.0 %) | 42 mg (1.7 %) | 1.25 g (32 %) |

WHAT WE CLAIM IS:

1. A process for preparing an $N^{\omega}$-trifluoroacetyl amino acid which comprises reacting a trifluoroacetic acid ester with a basic amino acid in an aqueous medium under an alkali condition.

2. The process of Claim 1, wherein said trifluoroacetic acid ester is an alkyl trifluoroacetate.

3. The process of Claim 1, wherein said basic amino acid is lysine or ornithine.

4. The process of Claim 1, wherein said alkali condition is a condition within the range of pH 8 to pH 11.